# EUROPEAN PATENT APPLICATION

(11) **EP 2 073 012 A1**
(43) Date of publication of application: **24.06.2009**
(21) Application number: 07123370.4
(22) Date of filing: 17.12.2007
(51) Int. Cl.: G01N 33/68

(54) **Method for the isolation and identification of ADP-ribosylated molecules**

(71) Applicant: Fondazione Telethon, 00154 Roma (IT); The European Molecular Biology Laboratory, 69117 Heidelberg (DE)
(72) Inventor: Corda, Daniela, 66034, S. Maria Imbaro (CH) (IT); Di Girolamo, Maria, 66034, S. Maria Imbaro (CH) (IT); Stilla, Annalisa, 66034, S. Maria Imbaro (CH) (IT); Marchegiani, Adriano, 66034, S. Maria Imbaro (CH) (IT); Dani, Nadia, 66034, S. Maria Imbaro (CH) (IT); Ladurner, Andreas, 69117 Heidelberg (DE); Till, Susanne, 69115, Heidelberg (DE)
(74) Representative: Capasso, Olga

(57) **Abstract**

The present invention relates to a method for the identification and/or isolation of ADP-ribosylated molecules, in particular mono-ADP-ribosylated proteins. The method makes use of molecules, namely polypeptides or proteins including *macro* domains as selective ligands for ADP- ribosylated molecules, and can be combined with known techniques for their identification.

## Description

### Field of the invention

The present invention relates to a method for the identification and/or isolation of ADP-ribosylated molecules, in particular mono-ADP-ribosylated proteins. The method makes use of molecules, namely polypeptides or proteins including *macro* domains as selective ligands for ADP- ribosylated molecules, and can be combined with known techniques for their identification.

### Background of the invention

Mono-ADP-ribosylation is a posttranslational modification catalyzed by bacterial toxins and eukaryotic enzymes that transfer the ADP-ribose moiety from β-NAD⁺ to various cellular acceptor proteins^{1,2}. Several bacterial ADP-ribosyltransferases (ARTs) are potent virulence factors. After translocation into the host cell, such factors modify eukaryotic proteins with high specificity, thus affecting their biological functions³. *Diphtheria* toxin (DT), produced by *Corynebacterium diphtheriae,* was the first bacterial ART family member to be identified⁴⁻⁶. Along with the related exotoxin A (ETA), which is produced by *Pseudomonas aeruginosa,* DT inhibits protein synthesis in intoxicated cells by catalyzing the ADP-ribosylation of diphthamide of elongation factor 2 (eEF2)⁴⁻⁶. Cholera toxin (CT) and *pertussis* toxin (PT) are two other well-characterized bacterial ARTs, which are produced by *Vibrio choler*α*e* and *Bordetell*α *pertussis* respectively, and which ADP-ribosylate the α subunits of trimeric G proteins (on arginine and cysteine residues, respectively) thus affecting their ability to transduce signals^{3,7,8}. Other toxins disrupt the cytoskeleton of mammalian cells by ADP-ribosylating either monomeric GTP-binding proteins of the Rho family or actin^{3,9-11}. In addition to these, it cannot be excluded that many more ADP-ribosylating toxins remain to be discovered¹²⁻¹⁴.

Importantly, endogenous, toxin-related ARTs have also been found in mammals, where they appear to mediate both intracellular and extracellular reactions^{1,2}. The latter are due to glycosylphosphatidylinositol (GPI)-anchored or secreted enzymes that can modify protein targets on the plasma membrane, including the P₂X₇ purinergic receptor, human neutrophil protein 1 (HNP1) and α7 integrin¹⁵⁻¹⁷. Intracellular, enzymatic mono-ADP-ribosylation modifies proteins with roles in cell signalling and metabolism, such as the chaperone GRP78/BiP, the β subunit of the heterotrimeric G proteins, and mitochondrial glutamate dehydrogenase (GDH)¹⁸⁻²¹. In addition, free ADP-ribose resulting from the turnover of cyclic ADP-ribose and ADP-ribose polymers or from hydrolase activities can bind to the cysteine and lysine residues of cellular proteins²²⁻²⁴. This non-enzymatic ADP-ribosylation is known as glycation and has been implicated in the physiopathology of age-related diseases^{25,26}. Finally, there are enzymes that reverse these posttranslational modifications and thus regulate cellular mono-ADP-ribosylation, such as cytosolic ADP-ribosylhydrolases and pyrophosphatases²⁷.

However, a systematic study of this modulation in intact cells has been prevented to date by the difficulties in the isolation of the mono-ADP-ribosylated substrates, due to a lack of specific antibodies or other specific ligands. Protein mono-ADP-ribosylation therefore remains little explored, despite its potential to have crucial roles in the regulation of complex biological processes, such as cell signalling, membrane trafficking, apoptosis and gene regulation^{1,2}

### Description of the invention

Thus the technical problem underlying the present invention is to set up a method for isolating and identifying ADP-ribosylated molecules in a biological sample, to check mono- or poly-ADP-ribosylation on targeted cellular proteins, i.e. by bacterial toxins or by endogenous enzymes, and to identify these targets, as well.

Recently, a protein module known as the *macro* domain was described to recognize monomeric and/or polymeric forms of the NAD metabolite ADP-ribose^{28,29}. The crystal structure of the ADP-ribose-*macro*-domain complex is also reported^{28,29}.

As disclosed at "http://www.expasy.org/prosite/PDOC51154", the *macro* domain (known also as Alpp, X domain, ADRP or *m*α*cro*-H2A domain) is a module of app. 180 amino acids which can bind ADP-ribose, an NAD metabolite or related ligands, such as but not limited to 2"- or 3"-*O*-acetyl-ADP-ribose (the Sir2 metabolites). The domain was described originally in association with ADP-ribose 1 "-phosphate (Appr-1"-P) processing activity (Alpp) of the yeast YBR022W protein [42]. The domain is called *macro* domain as it is the C-terminal domain of mammalian core histone macro-H2A [43,44]. *Macro* domain proteins are found in eukaryotes, in (mostly pathogenic) bacteria, in archaea and in ssRNA viruses, such as coronaviruses, Rubella and Hepatitis E viruses. In vertebrates the domain occurs e.g. in histone macroH2A, in predicted poly-ADP-ribose polymerases (PARPs) and in B aggressive lymphoma (BAL) protein.

The *macro* domain can be associated with catalytic domains, such as PARP (see <PDOC51059> Expasy Prosite http://www.expasy.org/prosite/PDOC51059), or sirtuin (See <PDOC50305> Expasy Prosite http://www.expasy.org/prosite/PDOC50305). The *macro* domain recognizes ADP-ribose or in some cases poly-ADP-ribose, which can be involved in ADP-ribosylation reactions that occur in important processes, such as chromatin biology, DNA repair and transcription regulation [28]. The human *macro*H2A1.1 *macro* domain binds an NAD metabolite O-acetyl-ADP-ribose [29]. The *macro* domain has been suggested to play a regulatory role in ADP-ribosylation, which is involved in inter- and intracellular signaling, transcriptional regulation, DNA repair pathways and maintenance of genomic stability, telomere dynamics, cell differentiation and proliferation, and necrosis and apoptosis.

The 3D structure of the *macro* domain has a mixed α/β fold of a mixed β sheet sandwiched between four helices (see <PDB:2BFQ> Pfam; http://www.sanger.ac.uk/Software/Pfam/). Several *macro* domains are shorter than the structure of AF1521 and lack either the first strand or the C-terminal helix 5. Well conserved residues form a hydrophobic cleft and cluster around the AF1521-ADP-ribose binding site [28, 29, 44, 45].

At present time, UniProtKB/Swiss-Prot and UniProtKB/TrEMBL protein databases refer to 1049 identified *macro* domains, 412 from viruses, 354 from bacteria, 222 from eukaryotes, 61 from Archaea.

A macro domain is also defined as any protein or translated DNA sequence which in BLASTP or tBLASTN searches (www.ncbi.nlm.nih.gov/BLAST/) shows >20% identity (called 'identities' in tBlastN) and/or >25% similarity (called 'positives' in tBlastN) over a contiguos stretch of at least 140 amino acid residues with the *macro* domains of *the A. fulgidus* Af1521, S. *cerevisiae* YBR022W, human macroH2A1.1. macroH2A1.2, macroH2A2 histones, in the SARS CoV virus, and any other protein whose structure resembles the crystal structures of the aforementioned (DALI score of 7 or higher; http://www.ebi.ac.uk/dali/)

A macro domain is also defined as any protein or translated DNA sequence that is listed with an Alpp or macro domain name in the 'DOMAIN' search of the SMART database (http://smart.embl-heidelberg.de/).

A macro domain is also defined as any protein or translated DNA sequence showing sequence homology to domains defined as Alpp or macro in the 'SEQUENCE ANALYSIS' search of the SMART database (http://smart.embl-heidelberg.be/).

A macro domain is also defined as any protein for which a NMR or X-ray crystal structure reveals a structural homology measured by a DALI Z-score of 7 (or higher) to thread the sequence homologs onto these structures (http://www.ebi.ac.uk/dali/).

An exemplificative list of proteins known to contain a *macro* domain includes:
- Mammalian histone *macro*-H2A.1 and *macro*-H2A.2, the latter with an alternatively spliced *macro* domain. These histones are enriched in the chromatin of inactive X-chromosomes and appear to have a role in transcriptional silencing [44, 29].
- Mammalian B aggressive lymphoma protein (BAL), which is a highly expressed risk factor in some aggressive lymphomas.
- Mammalian PARP-14 and PARP-15 proteins, with predicted PARP activity.
- Yeast YBR022W protein, which catalyzes the hydrolyzation of Appr-1"-P, a metabolite from tRNA splicing, to ADP-ribose and Pi.
- *Archaeoglobus fulgidus* AF1521 and related archaeal and bacterial proteins.
- Coronaviral papain-like proteinase (nsp3), in replicase polyprotein 1ab, which is responsible for cleavages at the N-terminus of replicase polyprotein.
- Rubella virus protease p150.
- Hepatitis E virus non-structural polyprotein.
- α virus non-structural protein 3.

The authors of the instant invention have surprisingly found that *macro* domains, as above defined and exemplified, bind to ADP-ribosylated molecules, in particular ADP-ribosylated proteins.

Then the present invention discloses that *macro* domains are powerful tools to specifically recognize mono- or poly-ADP-ribosylation on targeted cellular proteins. By combining the use of *macro* domains with known techniques, i.e. mass spectrometry, the method can be applied to many fields, as to the identification of targets within a host cell that are ADP-ribosylated by bacterial toxins or by endogenous enzymes.

The invention represents a unique tool to identify both endogenous and infection-induced ADP-ribosyl-proteomes in mammalian and other cell systems.

It is therefore an object of the instant invention a method for detecting an ADP-ribosylated molecule in a sample comprising the steps of:
a) incubating the sample with a molecule comprising at least one *macro* domain, said *macro* domain being able to bind ADP-ribose, an NAD metabolite or related ligands, under conditions that the ADP-ribosylated molecule, if present in the sample, binds to the *macro* domain, to form a complex;
b) separating the complex from the unbound molecule comprising at least one *macro* domain;
c) optionally detaching the ADP-ribosylated molecule from the complex;
d) detecting the ADP-ribosylated molecule.

It is intended that the skilled person may use any suitable macro domain capable of selectively bind to ADP-ribosylated molecules.

In a preferred embodiment the at least one *macro* domain able to bind ADP-ribose, an NAD metabolite or related ligands, has an amino acid sequence identity of at least n %, wherein n is a number comprised within 20 and 100, with any of the amino acid sequences over a region of at least 140 contiguous amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

In a more preferred embodiment the at least one *macro* domain has essentially any of the amino acid sequences over a region of at least contiguous 140 amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

In a particular aspect of the invention the at least one *macro* domain has essentially the sequence of SEQ ID No. 1 bearing a mutation G42E.

In a preferred embodiment the protein comprising at least one *macro* domain is labelled, and/or tagged with His or GST.

The method of detection of the invention may be advantageously used to detect ADP-ribosylated molecules as a protein, a peptide, a nucleic acid sequence, an intracellular ribosylated small molecule or an antibiotic. Preferably the ADP-ribosylated molecule is mono-ribosylated.

In a particular embodiment of the detection method, the sample is pretreated with a putative ADP-ribosylating agent. Preferably the sample is an environmental sample or a biological sample, i.e. cell, plant or tissue extract, preferably the sample is solubilized.

In a particular embodiment of the detection method the protein comprising at least one *macro* domain is bound to a solid support. i.e. a resin.

In a particular embodiment of the detection method, the sample is preincubated with the solid support alone to get a pre-clearing sample.

Preferably the pre-clearing sample is further preincubated with a mutated macro domain bearing a mutation that abrogates its binding to the to ADP-ribose, i.e the macro domain of sequence of SEQ ID No. 1 bearing a mutation G42E, to remove non specific molecule binding.

It is within the scope of the invention a kit, an array or a solid support, or similar technology, to perform the detection method above disclosed, comprising at least one *macro* domain as defined above. Biosensors comprising at least one *macro* domain as defined above are also within the scope of the invention.

The method of the invention is also useful to analyse kinetic interactions between a molecule comprising at least one ADP-ribose binding *macro* domain and a ADP-ribosylated molecule, namely a protein or a polypepetide carried out e.g. by Surface Plasmon Resonance.

It is a further object of the invention a method for isolating an ADP-ribosylated molecule from a biological sample comprising the steps of:
a) incubating the sample with a molecule comprising at least one ADP-ribose binding *macro* domain, said *macro* domain being able to bind ADP-ribose, an NAD metabolite or related ligands, under conditions that the ADP-ribosylated molecule, if present in the sample, binds to the ADP-ribose binding *macro* domain, to form a complex;
b) separating the complex from the unbound sample;
c) recovering the ADP-ribosylated molecule from the complex.

It is intended that the skilled person may use any suitable macro domain capable of selectively bind to ADP-ribosylated molecules.

In a preferred embodiment the at least one *macro* domain able to bind ADP-ribose, an NAD metabolite or related ligands, has an amino acid sequence identity of at least n %, wherein n is a number comprised within 20 and 100 with any of the amino acid sequences over a region of at least contiguous 140 amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

In a more preferred embodiment the at least one *macro* domain has essentially any of the amino acid sequences over a region of at least contiguous 140 amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

In a particular aspect of the invention the at least one *macro* domain has essentially the sequence of SEQ ID No. 1 bearing a mutation G42E.

In a preferred embodiment the protein comprising at least one *macro* domain is labelled, preferably is His or GST tagged.

The method of isolation of the invention may be advantageously used to detect ADP-ribosylated molecules as a protein, a peptide, a nucleic acid sequence, an intracellular ribosylated small molecule or an antibiotic. Preferably the ADP-ribosylated molecule is mono-ribosylated.

In a particular embodiment of the isolation method, the sample is pretreated with a putative ADP-ribosylating agent. Preferably the sample is an environmental sample or a biological sample, i.e. cell, plant or tissue extract, preferably the sample is solubilized.

In a particular embodiment of the isolation method the protein comprising at least one *macro* domain is bound to a solid support. i.e. a resin.

In a particular embodiment of the isolation method, the sample is preincubated with the solid support alone to get a pre-clearing sample.

Preferably the pre-clearing sample is further preincubated with a molecule having a mutated macro domain bearing a mutation that abrogates its binding to the to ADP-ribose, i.e. the macro domain of sequence of SEQ ID No. 1 bearing a mutation G42E, to remove non specific molecule binding,

It is within the scope of the invention a kit, an array or a solid support to perform the isolation method above disclosed, comprising at least one *macro* domain as above defined.

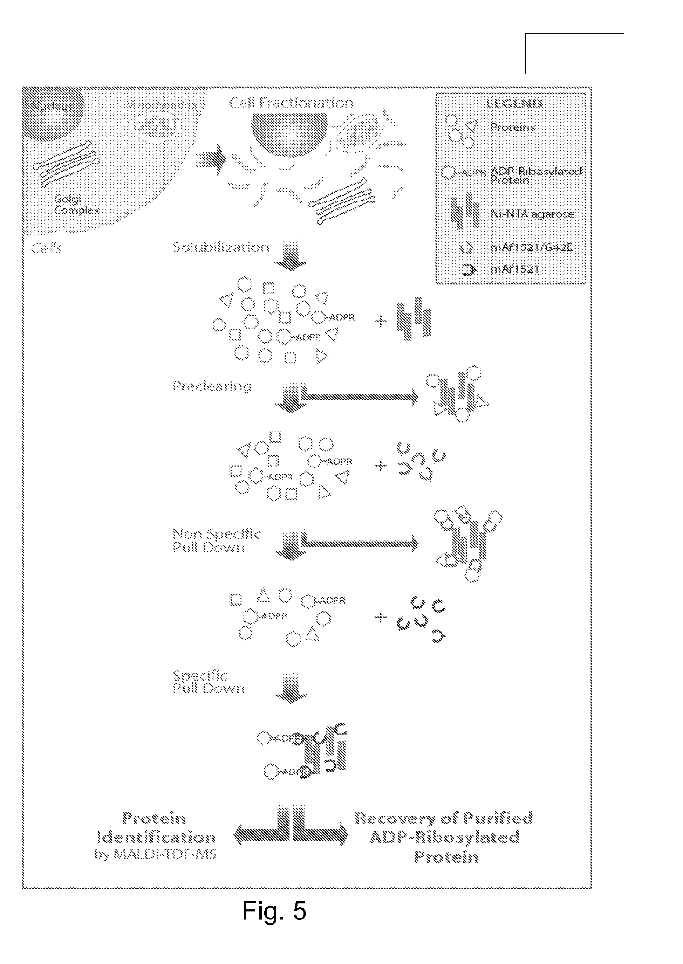

The present invention describes a powerful, affinity-based method for the analysis of endogenous and toxin-induced ADP-ribosylation of proteins in a sample, even in intact cells. In a particular embodiment, the method combines the use of a specific *macro-*domain module, *m*Af1521 (SEQ ID No. 1) (which binds ADP-ribosylated proteins potently and selectively) with MALDI-TOF-MS analysis (see scheme in following Fig. 5). While His₆-mAf1521 appears to be a very effective *macro* domain for the pulling down of ADP-ribosylated molecules from cells, the expert will appreciate that different *macro* domains and/or experimental conditions adjustments, i.e. detergents, incubation times, etc. are within the scope of the invention, for example when different cells or tissue extracts are to be analyzed. All the variants are included in the scope of the instant invention and comprise *macro* domains in labelled, tagged or radioactive forms.

The methods is of wide applicability to a large number of biological samples, which can all be analyzed following the principle of the protocol schematically summarized in following Figure 5. The method leads to the identification of previously unidentified proteins that can be regulated by ADP-ribosylation, usable to clarify the physiological and pathological roles of this reaction.

The authors have explored the use of the methodology in more than one way. The first was to purify known and novel ADP-ribosylated proteins and to separate modified and unmodified substrates. This is clearly shown for the mono-ADP-ribosylated β subunit, which can be eluted from resin-bound His₆-*m*Af1521 by some 100-fold excess of ADP-ribose, while the unmodified protein can easily be collected by immunoprecipitation of the unbound fraction. The ability to separate the modified from the unmodified protein represents a significant advantage in studies of the function of mono-ADP-ribosylated substrates, and it solves a technical aspect that to date could not be addressed by classical chromatography or immunoprecipitation.

The other specific use of the macro-domain method that the authors have investigated is in the field of bacterial toxins. There is significant interest in the role of mono-ADP-ribosylation in human pathology, as many bacterial toxins encode ARTs that disrupt cellular functions and affect human health^{3,6}. The number of known toxins with ART activity is growing¹²⁻¹⁴ and the new candidates require the characterization of their cellular targets. With the tools that have been available to date, this has not been straightforward: e.g., the characterization of the mammalian proteins that are ADP-ribosylated by the *Pseudomonas aeruginosa* toxin ExoT required the collection of silver-stained spots from 12 two-dimensional gels for MALDI-TOF analysis³⁶. This technical complexity in part explains why it has not been possible so far to define a compendium of all of the bacterial ART targets in mammalian host cells. The method of the present invention will greatly accelerate the analysis of this important class of toxins.

In perspective, while endogenous mono-ADP-ribosylation is believed to have important roles in cellular signalling and regulation, the enzymatic activities responsible for intracellular mono-ADP-ribosylation are only now beginning to be identified. The human genome, for example, encodes 17 so-called poly-ADP-ribose-polymerase-like genes. Many of these are, however, unlikely to carry out genuine enzymatic ADP-ribose polymer formation and some have been proposed to act as cellular mono-ADP-ribosyltransferases^{37,38}. Their cellular functions and targets remain unknown. Another NAD-using family of proteins, the sirtuins, encode enzymes that can deacetylate protein substrates, such as acetylated histones^{39,40}. The yeast member, silent information regulator 2 (Sir2) catalyzes the mono-ADP-ribosylation of the removed acetyl group³⁹; moreover, SirT6, has been shown to exhibit a mono-ADP-ribosyltransferase activity directed towards itself⁴¹ and SirT4 mono-ADP-ribosylates the mitochondrial GDH, thus repressing its activity²¹, resulting in the regulation of insulin secretion in pancreatic β cells. Moreover, to add to this complexity, several enzymes that catalyze the reverse reaction, the ADP-ribosyl hydrolases, have also been reported, although their specific roles and substrates also remain unknown. These examples give us a glimpse of the type of physiologically relevant information that can be gathered by fully exploring the endogenous mono-ADP-ribosylation machinery. The macro-domain-based methodology of the present invention allows a systematic study of the ADP-ribosyl-proteome in mammalian systems, and leads to the definition of the ADP-ribosylation-dependent pathways in physiology and pathology.

The present invention shall be disclosed in detail also by means of non limiting examples referring to the following figures.

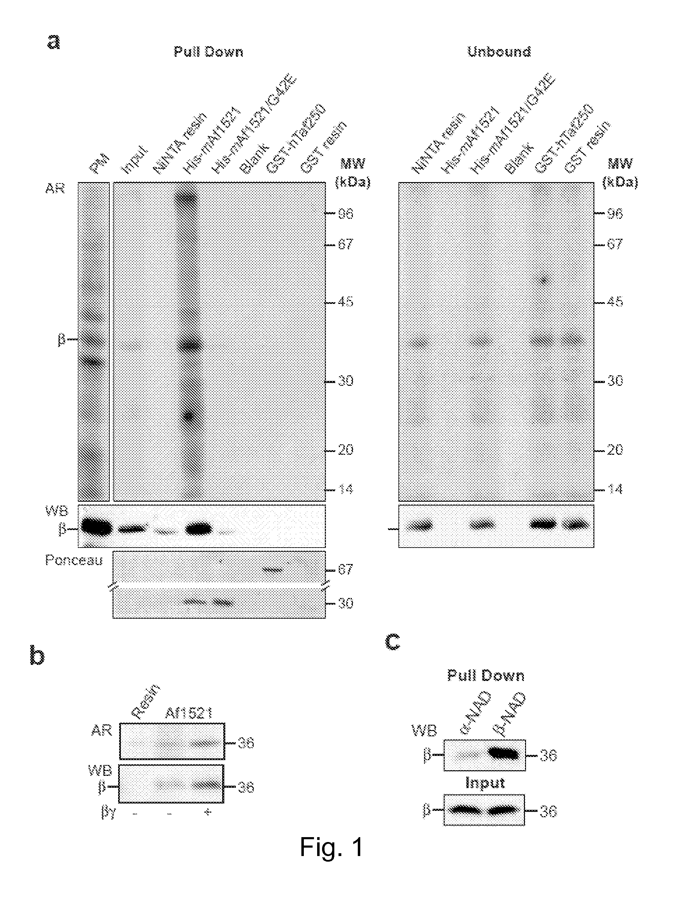

**Figure 1****.** Pull-down of the mono-ADP-ribosylated form of the β subunit from CHO cell plasma membranes by the *macro* modules. (a-c) Plasma-membrane proteins (10 µg) were ADP-ribosylated in the presence of [³²P]-NAD for 1 h at 37 °C, in the absence (a-d) or presence (c) of purified βγ dimer (250 ng) (see Methods), and then solubilised and pulled down with 200 pmole of the indicated *macro* modules. The pulled-down samples (a, c) and the unbound fractions (b; one-eighth of the total volume) were analysed by autoradiography (AR) and Western blotting (WB) with an antibody to the β subunit. The Ponceau Red staining (a, bottom) of the pulled-down samples shows that equivalent amounts of the different *macro* modules were used. (d) Plasma-membrane proteins (10 µg) were ADP-ribosylated in the presence of [³²P]-NAD for 12 h at 30 °C in the presence of unlabelled β-NAD and α-NAD (as indicated). Western blotting with an antibody to the β subunit of His₆-*m*Af1521-pulled down proteins and the corresponding input material. When β-NAD⁺ was replaced with α-NAD⁺, the residual labelling of substrates can be ascribed to the contaminating β-NAD⁺ present in the commercial α-NAD⁺ preparations. The data shown are representative of two to six experiments.

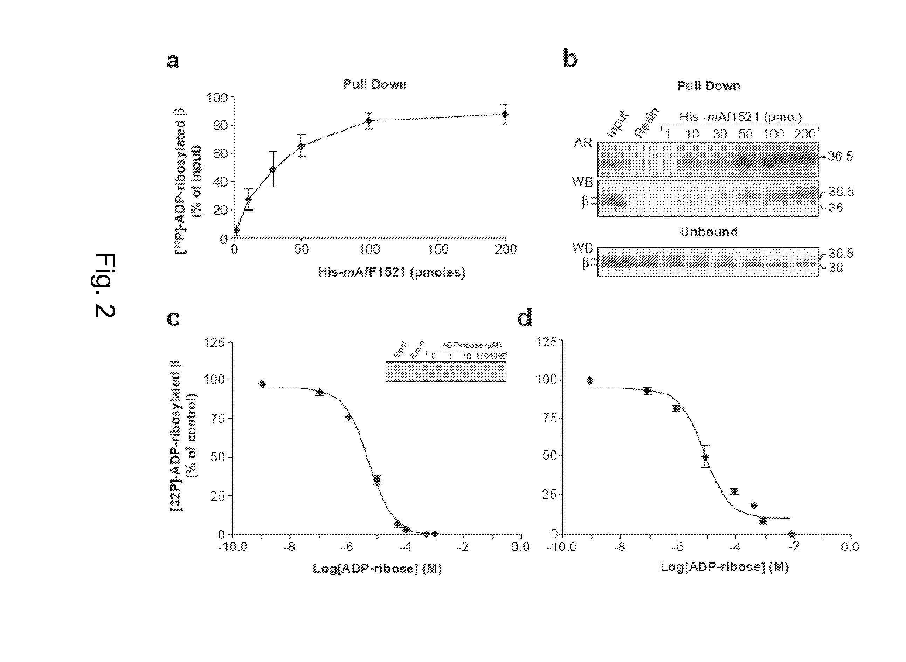

**Figure 2****.** Efficiency and reversibility of the binding of the ADP-ribosylated βγ dimer to His₆-*m*Af1521. CHO plasma membrane proteins (10 µg) and 250 ng purified βγ dimer were ADP-ribosylated in the presence of [³²P]-NAD for 12 h at 30 °C. Following solubilisation, they were pulled down with His₆-*m*Af1521. (a) Increasing concentrations of His₆-*m*Af1521 in the [³²P]-ADP-ribosylated β subunit pull-down, shown as percentage of the input of [³²P]-ADP-ribosylated β subunit. Data are means ±S.D. of 5 independent experiments. (b) As for (a), showing pulled-down samples and unbound fractions (one-eight of the total volume) from a representative experiment, as analysed by autoradiography (AR) and by Western blotting (WB) with an antibody against the β subunit that recognises both the unmodified (36 kDa) and the ADP-ribosylated (36.5 kDa) β subunit. The disappearance of the [³²P]-ADP-ribosylated β subunit from the unbound fraction was also followed in parallel (b, lower panel). (c) Pull down of the [³²P]-ADP-ribosylated β subunit after its incubation with 30 pmole His₆-*m*Af1521 in the presence of increasing concentrations of ADP-ribose (1 nM to 1 mM) ("competition" conditions). Data are expressed as percentages of [³²P]-ADP-ribosylated β subunit pulled down in the absence of ADP-ribose, and are means ±S.D. from 3 independent experiments. Insert: Autoradiography of the pulled-down samples from a representative experiment. (d) As (c), but with the addition of the ADP-ribose following the pull-down of the [³²P]-ADP-ribosylated β subunit by 30 pmole His₆-*m*Af1521 ("displacement" conditions). Data are means ±S.D. from 3 independent experiments. Curve fitting analyses in (c) and (d) were performed with GraphPad PRISM, giving ADP-ribose EC₅₀ values of 5 µM and 10 µM, respectively.

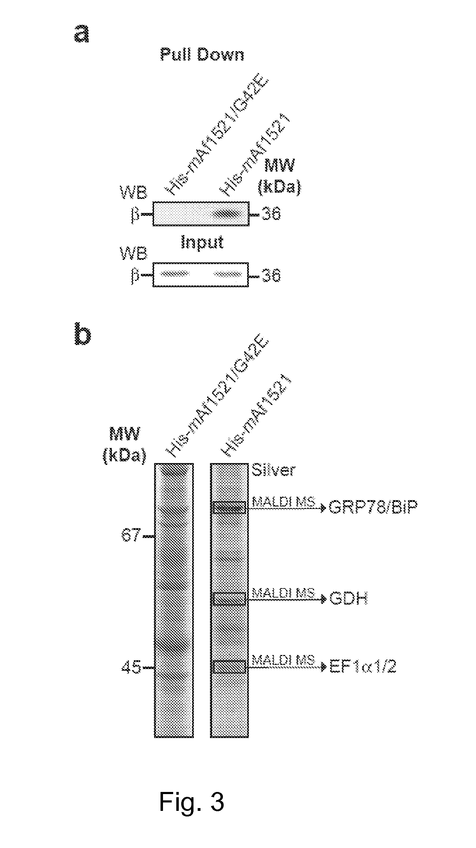

**Figure 3****.** His₆-*m*Af1521 pull-down of endogenous ADP-ribosyltransferases substrates. (a) Solubilised membrane proteins (2 mg) from HL60 cells were pulled down with 2 nmol of resin-bound His₆-*m*Af1521. The samples were analysed by SDS-PAGE followed by Western blotting (WB) with an antibody against the β subunit (see Methods). The relative content of the β subunit in the starting material in (a) was revealed by Western blotting (a, lower panel, WB). (b) Solubilised proteins (5 mg) from CHO post-nuclear fraction were pulled down with resin-bound His₆-*m*Af1521, resolved by SDS-PAGE, and visualised by Silver staining. The indicated bands were identified by MALDI-TOF-MS (see Methods and Table 1).

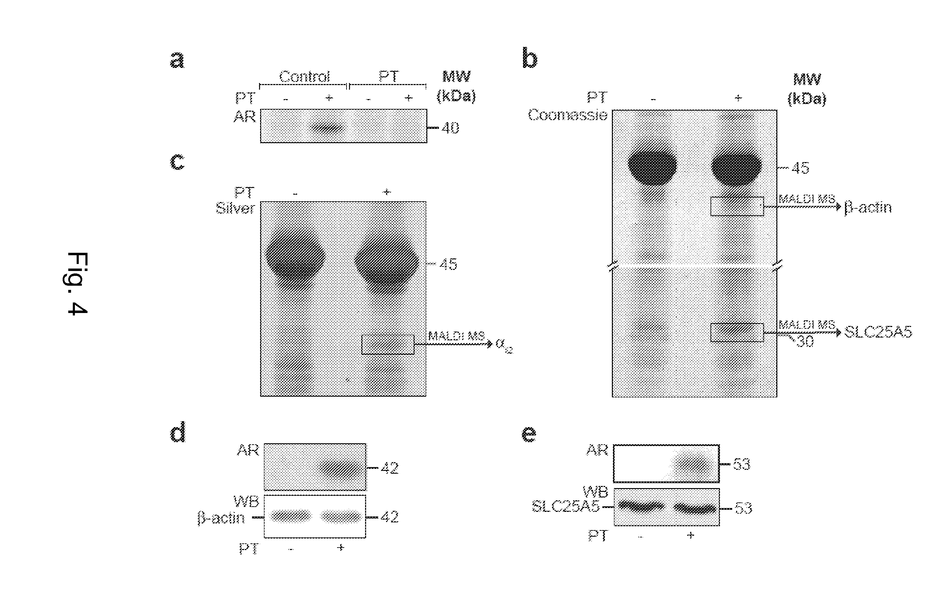

**Figure 4****.** GST-*m*Af1521 pull-down and substrate confirmation of PT ADP-ribosylated proteins from CHO cell plasma membranes. (a) Cells were untreated (control) or intoxicated with PT (PT), and their solubilised membrane proteins (25 µg) were ADP-ribosylated in the presence (+) of [³²P]-NAD and in the absence (-) and presence of PT (+) (see Methods). The samples were analysed by SDS-PAGE and visualized by autoradiography (AR). (b, c) Cells were untreated (-) or intoxicated with PT (+) and their solubilised membrane proteins (2 mg) were pulled down with 2 nmol GST-*m*Af1521. The samples were analysed by SDS-PAGE and visualised by colloidal Coomassie blue (b) or silver (c) staining. The indicated bands were identified by MALDI-TOF-MS (see Methods and Table 1). (d, e) Ten µg purified β-action (d) or GST-SLC25A5 (e) were ADP-ribosylated with [³²P]-NAD and in the absence (-) and presence (+) of PT in an *in vitro* assay (see Methods). The samples were analyzed by SDS-PAGE and visualized by autoradiography (AR) and Western blotting (WB) with antibodies to β-action (d) and GST (e).
**Figure 5****.** Schematic representation of the pull-down protocol using *m*Af1521. Following cell lysis and fractionation, the solubilised proteins are first pre-cleared using Ni-NTA resin alone. Non-specific resin/*m*Af1521 protein binding is then removed by the resin-bound His₆-*m*Af1521G42E mutant. Finally, the specific pull-down of the ADP-ribosylated proteins with resin-bound His₆-*m*Af1521 is followed by their separation by PAGE and either their recovery from the resin with free ADP-ribose, or their identification by mass spectrometry.

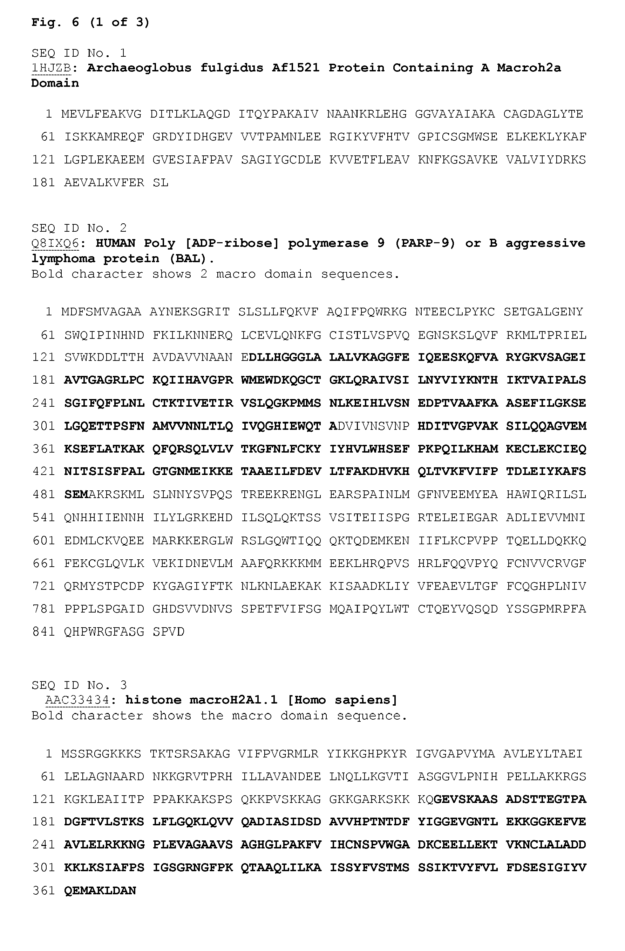

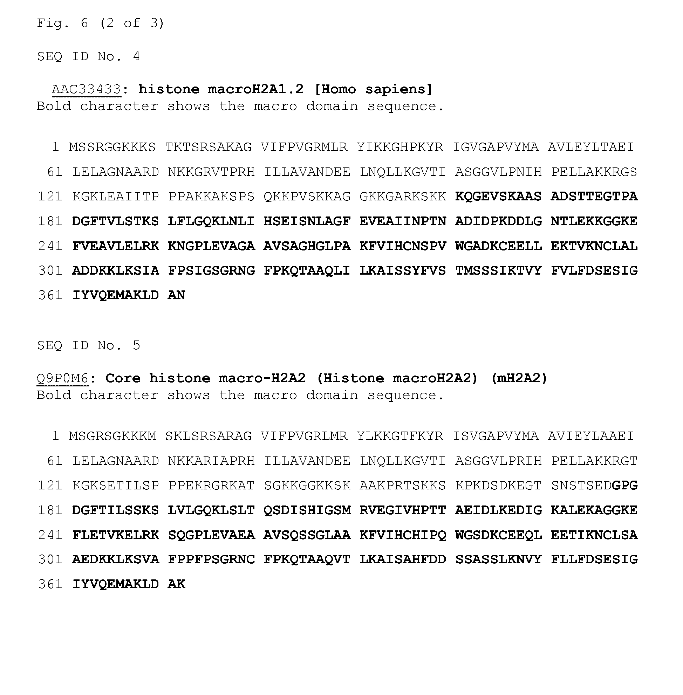

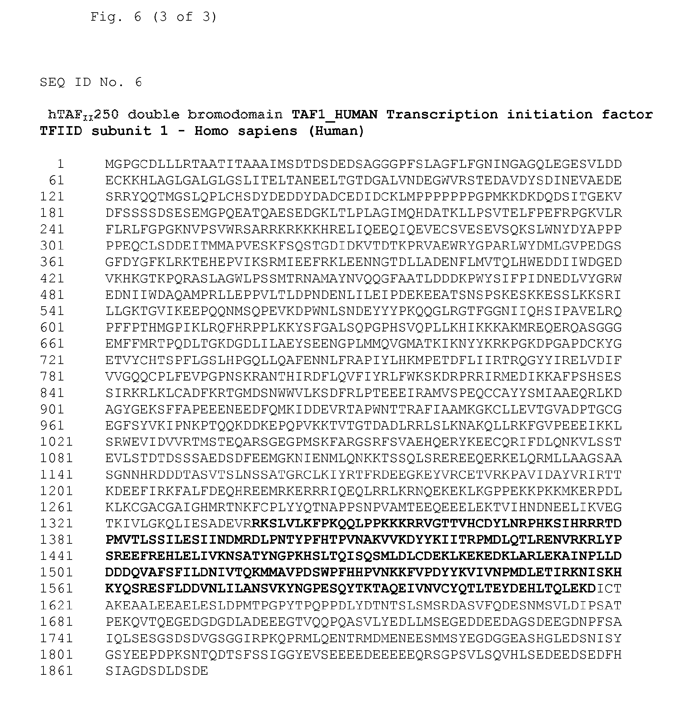

**Figure 6****.** Amino acid sequences of some *macro* domains and of proteins comprising them.

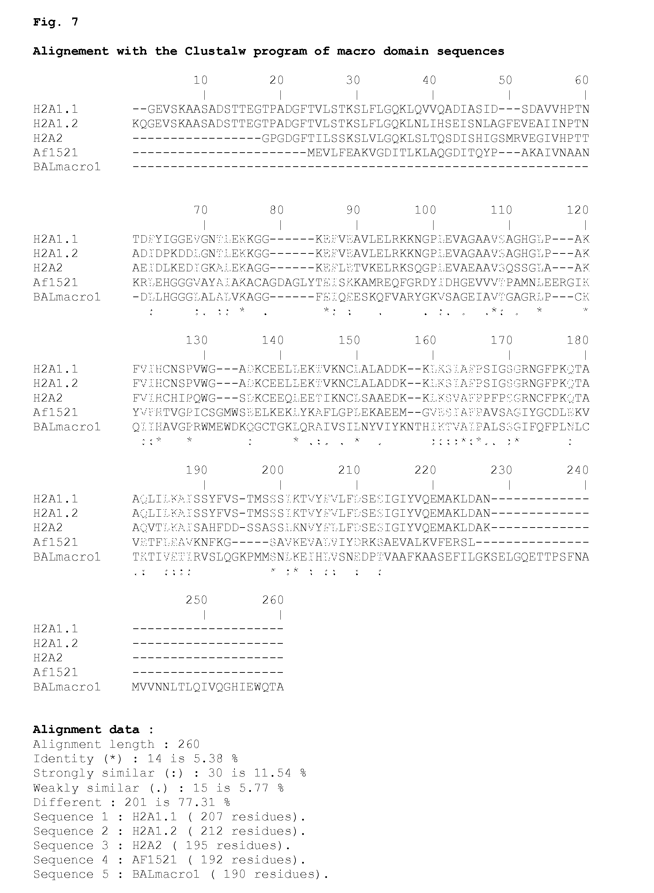

**Figure 7****.** Amino acid alignment according to the Clustlaw program of some *macro* domains, showing the homology of such domains due to clusters of identical or biochemically similar amino acids.

### Methods

### Expression and purification of macro modules.

The following *macro* modules were expressed and purified as previously described^{28, 29}: recombinant, N-terminus, His-tagged, full-length Af1521 wild-type (SEQ ID No. 1) and its G42E mutant; GST-tagged Af1521 wild-type and the GST-tagged hTaf1 (hTaf250) double bromodomain (aa. 1338-1617 of SEQ ID No. 6).

### Cell culture and fractionation.

The growth conditions for CHO and HL60 cells and the purification of plasma membranes were as previously described¹⁹. The total membrane and the postnuclear protein fractions were prepared from cells (10⁸ and 3 x 10⁸ cells per preparation, respectively) that were pelleted at 900x g for 10 min and washed three times with PBS. The cell pellets were resuspended either in 5 ml HES buffer (20 mM HEPES, pH 7.4, 1 mM EDTA, 250 mM sucrose and protease inhibitors) for the total membrane preparation, or in 1.5 ml lysis buffer (20 mM Tris-HCI, pH 7.4, 0.83 mM MgCl₂, 1 mM EDTA, 20 mM NaCl, 1 mM DTT and protease inhibitors) for the post-nuclear preparation; the cells were then lysed on ice by passage through a 25-gauge syringe. Unbroken cells and nuclei were removed by low-speed centrifugation (400x g for 5 min) to obtain the post-nuclear pellet. To obtain the total-membrane preparation, this post-nuclear was further centrifuged for 15 min at 16,000x g. The pellet here was resuspended in 20 mM HEPES, pH 7.4, containing 1 mM EDTA and protease inhibitors. Protein concentrations were determined using the Bio-Rad Protein Assay kit (Bio-Rad Laboratories), according to the manufacturer instructions.

### ADP-ribosylation assay and immunoblot analysis.

The ADP-ribosyltransferase activity was measured by following the incorporation of radioactive ADP-ribose into membrane proteins, as previously described¹⁹, with some modifications. Samples (10 µg plasma membranes) were incubated with 50 µl ADP-ribosylation buffer (50 mM potassium phosphate buffer (PFB), pH 7.4, 5 mM MgCl₂, 4 mM dithiothreitol (DTT), 10 µM GTPγS, 700 µM β-NAD⁺ and 4.5 µCi [³²P]-NAD (specific activity: 1,000 Ci/mmol)) at 37 °C for 60 min, unless otherwise specified. Under these conditions, 10% of the total β was mono-ADP-ribosylated; to obtain maximal modification (80%-100% of total β mono-ADP-ribosylated), the incubation was prolonged to 12 h at 30 °C. The *in-vitro* PT-catalyzed ADP-ribosylation was performed as previously described³⁴, using either total membranes or purified proteins (β-action or SLC25A5; Sigma and Abnova Corporation, respectively). The *in-vivo* PT-catalyzed ADP-ribosylation was performed by addition of 5 nM PT to cells for 15 h under controlled atmosphere conditions (5% CO₂/ 95% air) at 37 °C. The immunoblot analyses were performed as previously described³⁴, using an antiserum raised against the C-terminal peptide of the β subunit (Santa Cruz Biotechnology).

### Pull-down assay

Plasma membranes (10 µg for each sample) were ADP-ribosylated as described above, washed with 50 mM PFB, pH 7.4, to remove unbound NAD, and then centrifuged at 16.000x g for 15 min at 4 °C. The pellet was solubilised in 400 µl RIPA buffer (100 mM Tris/HCl, pH 7.5, 1% Igepal, 0.5% deoxycholate, 0.1% SDS, and protease inhibitors) with constant rotation for 30 min at 4 °C. The mixture was briefly sonicated on ice and clarified by centrifugation at 16.000x g for 45 min at 4 °C. The supernatant was incubated with the indicated concentrations of the specified *macro* modules for 2 h at room temperature on a rotating wheel, and then pulled down in an overnight incubation at 4 °C, with 50 µl of 50% slurry of Ni-NTA resin that had previously been equilibrated with RIPA buffer. Then the resin was washed three times with RIPA buffer plus 10 mM imidazole, and finally both the washed resin and the unbound fraction (one-eight of the total volume) were diluted in 50 µl Laemmli buffer, boiled and analysed by 10% SDS-PAGE. For the competition assays, the solubilised, ADP-ribosylated plasma membranes were simultaneously incubated with 30 pmole His₆-*m*Af1521 and increasing concentration of free cold ADP-ribose (1 nM to 10 mM) for 2 h at room temperature on a rotating wheel. For the displacement assay, plasma membranes were pulled down with His₆-*m*Af1521 as described above, and then incubated with increasing concentrations of free cold ADP-ribose (1 nM to 10 mM) for 4 h at room temperature on a rotating wheel. When the pull-down assay was performed using total membranes or post nuclear material, 2 mg protein for each sample were solubilised with 1.5 ml RIPA buffer as described above, and then absorbed with 100 µl of a 50% slurry of Ni-NTA beads for 2 h at 4 °C, for the pre-clearing step. The supernatant underwent two sequential pull downs, the first with 2 nmole *m*Af1521G42E and the second with 1 nmole His₆-*m*Af1521. The gels underwent colloidal Coomassie blue (blue code, Pierce) or silver staining, and the bands of interest were analysed by MALDI-TOF-MS.

Matrix-assisted laser desorption ionisation time-of-flight mass spectrometry (MALDI-TOF-MS).

The protein bands of interest from SDS-PAGE gels that were colloidal Coomassie blue or silver stained were excised and placed in 0.5 ml microcentrifuge tubes (Eppendorf, Hamburg, Germany). After washing, the cysteines were reduced with DTT and alkylated with iodoacetamide (Shevchenko and Shevchenko, 2001). The samples (gels) were digested *in situ* by incubation with sequencing-grade modified trypsin (Promega, Madison, WI, USA) in 40 mM ammonium bicarbonate, overnight at 37 °C under slight shaking on a thermo mixer. The reaction was stopped with 0.1% (v/v) TFA/H₂O (trifluoroacetic acid) for 15 min at 30 °C. The tryptic peptides were extracted with ZipTip C18 (Millipore Corp., Bedford, MA, USA) reverse-phase material, and directly eluted and crystallized in 50% (v/v) acetonitrile/H₂O saturated solution of β-cyan-4-hydroxy-cinnamic acid. The samples were analysed in a time-of-flight mass spectrometer (Reflex IV®, Bruker Daltonics, Bremen, Germany) equipped with a nitrogen laser with an emission wavelength of 337 nm. Mass spectra were acquiring in positive ion Reflectron-mode with delayed extraction and an accelerating voltage of 25 kV. External calibration was performed for each measurement using a mixture of seven standard peptides (average mass accuracy >20 ppm). All of the mass spectra were acquired using a minimum number of 250 laser shots. The spectra were internally calibrated with trypsin autolysis products. A database search with the monoisotopic peptide masses was performed against the NCBI non redundant database using the peptide search algorithm MASCOT (Matrix science, www.matrixscience.com). Mass tolerance of 100 ppm, single miss cleavage site per peptide fragments, carboamidomethyl modification of cysteine residues, and the optional presence of methionine oxidation were employed in the database search.

### Results

### Macro domain interactions with ADP-ribosylated proteins

The *macro* domains from both the human poly-(ADP-ribose)-polymerase (PARP) enzyme BAL9 (SEQ ID No. 2) and the archaeobacterial protein Af1521 from *Archaeoglobus fulgidus* (*m*Af1521, SEQ ID No. 1) recognize free ADP-ribose with high affinities and specificities (e.g., they bind NAD and adenosine with a >1000 fold lower affinity)²⁸. Despite their specificity, the *macro* domains of BAL9 and Af1521 also recognize the poly-ADP-ribose polymer *in vitro,* suggesting that the binding pocket can accommodate additional chemical groups beyond the core ADP-ribose²⁸. The authors therefore surmised that the *macro* domain module can also selectively bind mono-ADP-ribosylated proteins.

They first used a Ni²⁺-nitrilotriacetic acid (Ni-NTA)-resin pull-down assay based on the binding between the resin-bound, His-tagged *m*Af1521 (His₆-*m*Af1521; initially selected as the most efficient among other similar domains; see below) and a model ADP-ribosylation target, the well-characterized mono-ADP-ribosylated β subunit of G proteins^{19,30}. Plasma membranes were isolated from Chinese hamster ovary (CHO) cells and used as the source of the endogenously ADP-ribosylated βγ dimer (see Methods and ref. 19). His₆-*m*Af1521 was incubated with RIPA-buffer-solubilized plasma membranes from CHO cells in which the endogenous β subunit had been [³²P]-ADP-ribosylated, as previously described (see Methods and ref. 19), and its binding with the βγ subunit was assessed (Fig. 1). His₆-*m*Af1521 quantitatively retained the [³²P]-ADP-ribosylated β subunit, while the Ni-NTA resin alone did not. Similarly, an unrelated, glutathione-S-transferase GST-tagged hTaf1 (hTaf250) double bromodomain module that does not bind ADP-ribose²⁸, did not bind detectable levels of [³²P]-ADP-ribosylated β subunit (Fig. la; unbound protein in Fig. 1b). When the amount of the [³²P]-ADP-ribosylated β subunit to be loaded on the resin was increased by adding purified βγ dimer to the ADP-ribosylation assay (a condition in which βγ is known to associate with the plasma membrane and to be ADP-ribosylated^{19,31}), it was again quantitatively retained by His₆-*m*Af1521 (Fig. 1c). Finally, to ascertain that the interactions seen represented specific binding between *m*Af1521 and the mono-ADP-ribosylated form of the βγ dimer, the pull-down assay was carried out with plasma membranes that had been incubated with α-NAD (which, unlike β-NAD, cannot sustain enzymatic mono-ADP-ribosylation³¹). Under these conditions, His₆-*m*Af1521 did not retain the β subunit, as expected (Fig.1d).

To ascertain the specific role of the ADP-ribose binding pocket in the binding reaction, the authors also used a site-specific mutant (His₆-*m*Af1521/G42E) as a further control. This mutation from Gly to Glu is sufficient to abrogate the ADP-ribose binding of Af1521²⁸. As expected, this *m*Af1521 mutant failed to pull-down the [³²P]-ADP-ribosylated β subunit, confirming that the ADP-ribose binding pocket is essential for the interaction of *m*Af1521 with mono-ADP-ribosylated proteins (Fig. 1a).

Taken together, these data demonstrate that the ADP-ribose-binding *m*Af1521 interacts with the mono-ADP-ribosylated β subunit in a specific manner that depends on the β subunit ADP-ribosylation state. Notably, other *macro* domains, including His₆-*m*H2A2 (SEQ ID No. 5) and His₆-*m*H2A1.1²⁹ (SEQ ID No. 3), also supported the pull-down of ADP-ribosylated βγ, although with a lower efficiency than His₆-*m*Af1521. One *macro* module (His₆-*m*H2A1.2 SEQ ID No. 4) that does not have the ability to bind ADP-ribose, did not bind ADP-ribosylated βγ.

Thus ADP-ribose-binding *macro* domains represent valuable tools for the study of endogenous ADP-ribosylation of proteins.

### Efficiency and reversibility of the binding of ADP-ribosylated proteins to mAf1521

Affinity purification procedures ideally require that the binding between the proteins to be isolated and the affinity resin be both efficient and reversible. To investigate these characteristics in our system, 10 µg of CHO cell plasma membranes enriched with 250 ng of purified βγ dimer were first ADP-ribosylated in the presence of [³²P]-NAD (see Methods and ref. 19, 31). The amount of [³²P]-ADP-ribosylated β subunit that was pulled down using increasing concentrations of HiS₆-*m*Af1521 was then evaluated by autoradiography and Western blotting (Fig. 2a, b; unbound fractions shown as control in Fig. 2b, lower panel). With the 100% [³²P]-ADP-ribosylated β subunit input of Figure 2a representing -4 pmol³¹, 50% was retained by -25 pmol His₆-*m*Af1521, with a -20-fold molar excess of resin-bound His₆-*m*Af1521 being sufficient to nearly completely (∼90%) absorb the ADP-ribosylated βγ.

The authors also examined the reversibility of the binding of the [³²P]-ADP-ribosylated β subunit to His₆-*m*Af1521. Here, a range of ADP-ribose concentrations (1 nM to 10 mM) and 30 pmol His₆-*m*Af1521 were used, either by adding the His₆-*m*Af1521 and

ADP-ribose to the labelled, solubilised, CHO plasma membranes simultaneously ("competition"; Fig. 2c), followed by the resin pull down, or by initially pulling down the [³²P]-ADP-ribosylated β subunit with the resin-bound His₆-*m*Af1521 and then adding the ADP-ribose ("displacement"; Fig. 2d; see Methods for further details). This demonstrated that ADP-ribose can displace the [³²P]-ADP-ribosylated β subunit from the resin with EC₅₀ values of 5 µM and 10 µM, respectively. On the basis of using 100 µM ADP-ribose (∼400 pmol under these incubation conditions) to displace all of the His₆-*m*Af1521-bound [³²P]-ADP-ribosylated β subunit (∼4 pmol; Fig. 2a, c, d), a -100-fold excess of ADP-ribose is able to displace the immobilized ADP-ribosylated β subunit from the His₆-*m*Af1521-resin complex. This thus indicates that our *macro-*domain-based procedure can yield highly pure and intact ADP-ribosylated proteins in solution.

### Purification and identification of substrates of endogenous intracellular ART activities by macro domains

While *m*Af1521 binds the β subunit of G proteins in its mono-ADP-ribosylated form, intracellular mono-ADP-ribosylation in mammals modifies other proteins that have important roles in cell signalling and metabolism, including among those best characterized the chaperone GRP78/BiP and GDH^{18-21,32}.

The authors next evaluated whether macro-domain-based pull-down assays coupled to MALDI-TOF-MS analysis can be used to identify other proteins that carry an endogenous ADP-ribosyl modification under resting conditions in an intact-cell model. Membrane fractions isolated from CHO and HL60 cells were used in parallel, where they were first subjected to a non-specific pull-down step using the His₆-tagged *m*Af1521/G42E mutant; this thus initially eliminated proteins that bound to the resin or to the *macro* domain in an ADP-ribose-independent manner (Fig. 3a, b). Next, the unbound material underwent a second pull-down assay with wild-type His₆-*m*Af1521, to specifically retain the ADP-ribosylated proteins (Fig. 3a, b).

This two-steps procedure resulted in the separation of more than 40 visible bands in well-resolved gels, all representing potential ADP-ribosylated substrates (Fig. 3b and data not shown). Three of the 10 major bands from CHO cells were identified by MALDI-TOF-MS analysis as GRP78/BiP, GDH and the elongation factor α1/2 (Table 1). Similar results were obtained with solubilised total membranes from HL60 cells. These four proteins are well-known substrates of enzymatic ADP-ribosylation and provide the "proof of principle" that our two-step procedure can successfully isolate different ADP-ribosylated proteins.

An additional interesting feature of the macro-domain recognition of ADP-ribosylated substrates is its independence of the nature of the modified amino acid. The *m*Af1521 not only binds the G-protein β subunit that the authors have shown to be modified on arginine¹⁹, but also GDH that is modified on cysteine²⁰, eEF2 on diphthamide^{6,33}, and

GRP78/BiP on a yet-to-be-identified amino acid, clearly indicating that the recognition of the ADP-ribose is independent of the modified amino acid. This highlights the potential of our methodology and its relevance for the definition of the role of the ADP-ribosylation reaction.

In summary, these data show that a two-step procedure with first mutant and then wild-type *m*Af1521 is very effective in isolating substrates of endogenous ART activities, which should now lead to the identification of novel substrates of the ADP-ribosylation reaction.

### Use of the macro module to identify known and novel cellular targets of bacterial ART toxins

The authors checked whether the method can be applied to identify substrates of bacterial toxins that display ART activity, including potentially unidentified novel targets. They focused their attention on the cellular substrates of pertussis toxin (PT), a cysteine-specific ART. For this, they used CHO cells that were either left untreated (control) or were intoxicated with PT (5 nM for 15 h; see Methods). First, to verify that the αᵢ subunit of the heterotrimeric G proteins was indeed fully modified by the *in-vivo* treatment with PT, membranes from control and intoxicated cells were analyzed in an *in-vitro* ADP-ribosylation assay using PT as enzyme and with radiolabelled NAD, according to established procedures (see Methods and ref. 34). While in control membranes αᵢ was radiolabelled by PT, in the intoxicated membranes this Gᵢ subunit was not radiolabelled, confirming that it had already been fully modified *in vivo* by the PT pretreatment and was therefore unavailable for *in-vitro* labelling (Fig. 4a).

These PT-treated membranes were next subjected to the macro-domain pull down protocol: they were solubilized, and underwent pre-clearing with the resin, and then the pull-down with GST-*m*Af1521 (Fig. 4b). Colloidal Coomassie blue staining of the resulting gels revealed two main proteins that were only present in the PT-treated samples, with molecular masses of 42 kDa and 33 kDa (Fig. 4b). These were isolated and analyzed by MALDI-TOF-MS, and were identified as β-action and a member of the mitochondrial solute-carrier family 25 (SLC25A5), respectively (Table 1). As the expected PT substrate, i.e. the 40-kDa G-protein αᵢ subunit, was too scarce to be revealed by colloidal Coomassie blue staining, parallel samples were analyzed by silver staining (Fig. 4c). Here, a band in the PT-treated samples with a molecular mass of 40 kDa was indeed revealed and identified by MALDI-TOF-MS analysis as the G-protein αᵢ₂ subunit (Table 1). In confirmation of these findings, the same three PT-ADP-ribosylated bands, i.e., the G-protein αᵢ subunit, β-action and SLC25A5, were isolated from HL60 cell membranes following a procedure parallel to that used for CHO cells (data not shown). Finally, to further substantiate the modification of these novel PT substrates, an *in-vitro* assay using purified β-action and SLC25A5 as substrates, PT as the ART (5 nM for 1 h), and radiolabelled β̃NAD was performed. Under these conditions, both β-action and SLC25A5 were radiolabelled, clearly indicating that they are indeed substrates of PT-dependent ADP-ribosylation (Fig. 4d, e).

Thus, using this *macro*-domain-mass-spectrometry protocol, the authors have identified β-action and SLC25A5 as novel cellular targets of PT. Interestingly, it has already been proposed that actin can be regulated by ADP-ribosylation by cellular enzymes (although only in an *in-vitro* analysis)³⁵, whereas the modification of the SLC25A5 mitochondrial transporter is completely novel. The pathological and mechanicistic significance of the modification of these two novel targets in infected eukaryotic host cells now needs to be determined.

**Table 1. Maldi- TOF-MS protein identificαtion**

| **n. NCBI** | **Name** | **Score*** | **n. Matched Peptide** | **Sequence Coverage** % | **MW (dalton)** |
|---|---|---|---|---|---|
| 22094075 | solute carrier family 25, member 5 | 129 | 17 | 51 | 33138 |
| 77386129 | Gai2 | 112 | 10 | 27 | 40989 |
| 71620 | Actin-b | 92 | 9 | 42 | 42066 |
| 121570 | GRP 78 | 101 | 12 | 22 | 72505 |
| 1220484 | Elongation factor-1a | 109 | 13 | 37 | 50460 |
| 6680027 | Glutamate 1 dehydrogenase 1 | 134 | 18 | 35 | 61640 |

| | | | | | |
|---|---|---|---|---|---|
| *Protein score greater than 65 are significant (P< 0.05). See Methods for details | | | | | |

### References

1. Corda, D. & Di Girolamo, Embo J 22, 1953-8 (2003).
2. Seman, M., Adriouch, S., Haag, F. & Koch-Nolte, F. Curr Med Chem 11, 857-72 (2004).
3. Krueger, K. M. & Barbieri, J. T. Clin Microbiol Rev 8, 34-47. (1995).
4. Honjo, T., Nishizuka, Y. & Hayaishi, O. J Biol Chem 243, 3553-5 (1968).
5. Collier, R. J. & Cole, H. A. Science 164, 1179-81 (1969).
6. Yates, S. P. et al., Trends Biochem Sci 31, 123-33 (2006).
7. Cassel, D. & Pfeuffer, T. Proc Natl Acad Sci U S A 75, 2669-73 (1978).
8. Katada, T. & Ui, M. Proc Natl Acad Sci U S A 79, 3129-33. (1982).
9. Henriksson, M. L. et al. Biochem J 22 (2002).
10. Aktories, K. & Hall, A. Trends Pharmacol Sci 10, 415-8. (1989).
11. Vandekerckhove, J. et al., J Biol Chem 263, 696-700. (1988).
12. Pallen, M. J. et al., Trends Microbiol 9, 302-7; discussion 308. (2001).
13. Wilde, C. et al., J Biol Chem 276, 9537-42. (2001).
14. Masignani, V. et al. Mol Microbiol 50, 1055-67 (2003).
15. Seman, M. et al. Immunity 19, 571-82 (2003).
16. Paone, G. et al. Proc Natl Acad Sci U S A 99, 8231-5. (2002).
17. Zolkiewska, A. & Moss, J. J Biol Chem 268, 25273-6. (1993).
18. Leno, G. H. & Ledford, B. E. Eur. J. Biochem. 186, 205-11. (1989).
19. Lupi, R., Corda, D. & Di Girolamo, M. J Biol Chem 275, 9418-24. (2000).
20. Herrero-Yraola, A. et al. Embo J 20, 2404-12. (2001).
21. Haigis, M. C. et al. Cell 126, 941-54 (2006).
22. McDonald, L. J. et al., J. Biochemistry 31, 11881-7. (1992).
23. Cervantes-Laurean, D. et al., J Biol Chem 270, 7929-36. (1995).
24. Grimaldi, J. C. et al. J Immunol 155, 811-7. (1995).
25. Kreimeyer, A. et al., J Biol Chem 259, 890-6. (1984).
26. Jacobson, E. L. et al., Adv Exp Med Biol 419, 371-9 (1997).
27. Moss, J., Zolkiewska, A. & Okazaki, I. Adv Exp Med Biol 419, 25-33 (1997).
28. Karras, G. I. et al. Embo J 24, 1911-20 (2005).
29. Kustatscher, G. et al., Nat Struct Mol Biol 12, 624-5 (2005).
30. Di Girolamo, M., Dani, N., Stilla, A. & Corda, D. Febs J 272, 4565-75 (2005).
31. Lupi, R. et al. Biochem J 30 (2002).
32. Laitusis, A. L. et al., J Biol Chem 274, 486-93. (1999).
33. Fendrick, J. L. et al., Eur J Biochem 205, 25-31. (1992).
34. Di Girolamo, M. et al., J Biol Chem 267, 17397-403 (1992).
35. Terashima, M. et al., Biochem Mol Biol Int 47, 615-20. (1999).
36. Sun, J. & Barbieri, J. T. J Biol Chem 278, 32794-800 (2003).
37. Otto, H. et al. BMC Genomics 6, 139 (2005).
38. Ame, J. C., Spenlehauer, C. & de Murcia, G. Bioessays 26, 882-93 (2004).
39. Tanner, K. G. et al., Proc Natl Acad Sci U S A 97, 14178-82. (2000).
40. Frye, R. A. Biochem Biophys Res Commun 273, 793-8. (2000).
41. Liszt, G. et al., J Biol Chem 280, 21313-20 (2005).
42. Martzen M.R., et al. Science 286:1153-1155(1999).
43. Aravind L. Trends Biochem. Sci. 26:273-275(2001).
44. Allen M.D., et al. J. Mol. Biol. 330:503-511(2003).
45. Egloff M.P., et al. J. Virol. 80:8493-8502(2006).

## Claims

1. A method for detecting an ADP-ribosylated molecule in a sample comprising the steps of:
a) incubating the sample with a molecule comprising at least one *macro* domain, said *macro* domain being able to bind ADP-ribose, an NAD metabolite or related ligands, under conditions that the ADP-ribosylated molecule, if present in the sample, binds to the *macro* domain, to form a complex;
b) separating the complex from the unbound molecule comprising at least one *macro* domain;
c) optionally detaching the ADP-ribosylated molecule from the complex;
d) detecting the ADP-ribosylated molecule.

2. The method according to claim 1 wherein the at least one *macro* domain able to bind ADP-ribose, an NAD metabolite or related ligands, has an amino acid sequence identity of at least n %, wherein n is a number comprised within 20 and 100 with any of the amino acid sequences over a region of at least contiguous 140 amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

3. The method according to claim 2 wherein the at least one *macro* domain has essentially any of the amino acid sequences over a region of at least contiguous 140 amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

4. The method according to any of previous claims wherein the molecule comprising at least one *macro* domain is labelled.

5. The method according to any of previous claims wherein the molecule comprising at least one *macro* domain is His or GST tagged.

6. The method according to any of previous claims wherein the ADP-ribosylated molecule is a protein, a peptide, a nucleic acid sequence, an intracellular ribosylated small molecule or an antibiotic.

7. The method according to any of previous claims wherein the ADP-ribosylated molecule is mono-ribosylated.

8. The method according to any of previous claims wherein the sample is pretreated with a putative ADP-ribosylating agent.

9. The method according to any of previous claims wherein the sample is an environmental sample or a biological sample.

10. The method according to claim 9 wherein the sample is solubilized.

11. The method according to claims 9 or 10 wherein the sample is a cell, plant or tissue extract.

12. The method according to any of previous claims wherein the protein comprising at least one *macro* domain is bound to a solid support.

13. The method according to claim 12 wherein the support is a resin.

14. The method according to claims 12 or 13 wherein the sample is preincubated with the solid support alone to get a pre-clearing sample.

15. The method according to claim 14 wherein the pre-clearing sample is further preincubated with a mutated *macro* domain bearing a mutation that abrogates its binding to the to ADP-ribose to remove non specific molecule binding.

16. The method according to claim 15 wherein when the mutated *macro* domain has essentially the sequence of SEQ ID No. 1 bearing a mutation G42E.

17. A kit, an array or a solid support to perform the detection method according to any of claims from 1 to 16 comprising at least one *macro* domain as defined in claims 1 to 5.

18. A method for isolating an ADP-ribosylated molecule from a biological sample comprising the steps of:
a) incubating the sample with a molecule comprising at least one ADP-ribose binding *macro* domain, said *macro* domain being able to bind ADP-ribose, an NAD metabolite or related ligands, under conditions that the ADP-ribosylated molecule, if present in the sample, binds to the ADP-ribose binding *macro* domain, to form a complex;
b) separating the complex from the unbound sample;
c) recovering the ADP-ribosylated molecule from the complex.

19. The method according to claim 18 wherein the at least one *macro* domain able to bind ADP-ribose, an NAD metabolite or related ligands, has an amino acid sequence identity of at least n %, wherein n is a number comprised within 20 and 100 with any of the amino acid sequences over a region of at least contiguous 140 amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

20. The method according to claim 19 wherein the at least one *macro* domain has essentially any of the amino acid sequences over a region of at least contiguous 140 amino acids belonging to the following group: aa. 1-181 of SEQ ID No. 1; aa. 162-331 of SEQ ID No. 2; aa. 341-483 of SEQ ID No. 2; aa. 163-369 of SEQ ID No. 3; aa. 161-372 of SEQ ID No. 4; aa. 178-372 of SEQ ID No. 5.

21. The method according to any of previous claims 18 to 20 wherein the molecule comprising at least one *macro* domain is labelled.

22. The method according to any of previous claims 18 to 21 wherein the molecule comprising at least one *macro* domain is His or GST tagged.

23. The method according to any of previous claims 18 to 22 wherein the ADP-ribosylated molecule is a protein, a peptide, a nucleic acid sequence, an intracellular ribosylated small molecule or an antibiotic.

24. The method according to any of previous claims 18 to 23 wherein the ADP-ribosylated molecule is mono-ribosylated.

25. The method according to any of previous claims 18 to 24 wherein the sample is pretreated with a putative ADP-ribosylating agent.

26. The method according to any of previous claims 18 to 25 wherein the sample is an environmental sample or a biological sample.

27. The method according to claim 26 wherein the sample is solubilized.

28. The method according to claims 26 or 27 wherein the sample is a cell, plant or tissue extract.

29. The method according to any of previous claims 18 to 28 wherein the protein comprising at least one *macro* domain is bound to a solid support.

30. The method according to claim 29 wherein the support is a resin.

31. The method according to claims 29 or 30 wherein the sample is preincubated with the solid support alone to get a pre-clearing sample.

32. The method according to claim 31 wherein the pre-clearing sample is further preincubated with a mutated *macro* domain bearing a mutation that abrogates its binding to the to ADP-ribose to remove non specific molecule binding.

33. The method according to claim 32 wherein when the mutated *macro* domain has essentially the sequence of SEQ ID No. 1 bearing a mutation G42E.

34. A kit, an array or a solid support to perform the isolation method according to any of claims from 18 to 33 comprising at least one *macro* domain as defined in claims 18 to 22.


SEQUENCE LISTING

<110>  Fondazione Telethon
       European Molecular Biology Laboratory

<120>  Method for the isolation and identification of ADP-ribosylated
       molecules

<130>  BE99508

<160>  6

<170>  PatentIn version 3.3

<210>  1
<211>  192
<212>  PRT
<213>  Archaeoglobus fulgidus

<400>  1

Met Glu Val Leu Phe Glu Ala Lys Val Gly Asp Ile Thr Leu Lys Leu
1               5                   10                  15

Ala Gln Gly Asp Ile Thr Gln Tyr Pro Ala Lys Ala Ile Val Asn Ala
            20                  25                  30

Ala Asn Lys Arg Leu Glu His Gly Gly Gly Val Ala Tyr Ala Ile Ala
        35                  40                  45

Lys Ala Cys Ala Gly Asp Ala Gly Leu Tyr Thr Glu Ile Ser Lys Lys
    50                  55                  60

Ala Met Arg Glu Gln Phe Gly Arg Asp Tyr Ile Asp His Gly Glu Val
65                  70                  75                  80

Val Val Thr Pro Ala Met Asn Leu Glu Glu Arg Gly Ile Lys Tyr Val
                85                  90                  95

Phe His Thr Val Gly Pro Ile Cys Ser Gly Met Trp Ser Glu Glu Leu
            100                 105                 110

Lys Glu Lys Leu Tyr Lys Ala Phe Leu Gly Pro Leu Glu Lys Ala Glu
        115                 120                 125

Glu Met Gly Val Glu Ser Ile Ala Phe Pro Ala Val Ser Ala Gly Ile
    130                 135                 140

Tyr Gly Cys Asp Leu Glu Lys Val Val Glu Thr Phe Leu Glu Ala Val
145                 150                 155                 160

Lys Asn Phe Lys Gly Ser Ala Val Lys Glu Val Ala Leu Val Ile Tyr
                165                 170                 175

Asp Arg Lys Ser Ala Glu Val Ala Leu Lys Val Phe Glu Arg Ser Leu
            180                 185                 190

<210>  2
<211>  854
<212>  PRT
<213>  Homo sapiens

<400>  2

Met Asp Phe Ser Met Val Ala Gly Ala Ala Ala Tyr Asn Glu Lys Ser
1               5                   10                  15

Gly Arg Ile Thr Ser Leu Ser Leu Leu Phe Gln Lys Val Phe Ala Gln
            20                  25                  30

Ile Phe Pro Gln Trp Arg Lys Gly Asn Thr Glu Glu Cys Leu Pro Tyr
        35                  40                  45

Lys Cys Ser Glu Thr Gly Ala Leu Gly Glu Asn Tyr Ser Trp Gln Ile
    50                  55                  60

Pro Ile Asn His Asn Asp Phe Lys Ile Leu Lys Asn Asn Glu Arg Gln
65                  70                  75                  80

Leu Cys Glu Val Leu Gln Asn Lys Phe Gly Cys Ile Ser Thr Leu Val
                85                  90                  95

Ser Pro Val Gln Glu Gly Asn Ser Lys Ser Leu Gln Val Phe Arg Lys
            100                 105                 110

Met Leu Thr Pro Arg Ile Glu Leu Ser Val Trp Lys Asp Asp Leu Thr
        115                 120                 125

Thr His Ala Val Asp Ala Val Val Asn Ala Ala Asn Glu Asp Leu Leu
    130                 135                 140

His Gly Gly Gly Leu Ala Leu Ala Leu Val Lys Ala Gly Gly Phe Glu
145                 150                 155                 160

Ile Gln Glu Glu Ser Lys Gln Phe Val Ala Arg Tyr Gly Lys Val Ser
                165                 170                 175

Ala Gly Glu Ile Ala Val Thr Gly Ala Gly Arg Leu Pro Cys Lys Gln
            180                 185                 190

Ile Ile His Ala Val Gly Pro Arg Trp Met Glu Trp Asp Lys Gln Gly
        195                 200                 205

Cys Thr Gly Lys Leu Gln Arg Ala Ile Val Ser Ile Leu Asn Tyr Val
    210                 215                 220

Ile Tyr Lys Asn Thr His Ile Lys Thr Val Ala Ile Pro Ala Leu Ser
225                 230                 235                 240

Ser Gly Ile Phe Gln Phe Pro Leu Asn Leu Cys Thr Lys Thr Ile Val
                245                 250                 255

Glu Thr Ile Arg Val Ser Leu Gln Gly Lys Pro Met Met Ser Asn Leu
            260                 265                 270

Lys Glu Ile His Leu Val Ser Asn Glu Asp Pro Thr Val Ala Ala Phe
        275                 280                 285

Lys Ala Ala Ser Glu Phe Ile Leu Gly Lys Ser Glu Leu Gly Gln Glu
    290                 295                 300

Thr Thr Pro Ser Phe Asn Ala Met Val Val Asn Asn Leu Thr Leu Gln
305                 310                 315                 320

Ile Val Gln Gly His Ile Glu Trp Gln Thr Ala Asp Val Ile Val Asn
                325                 330                 335

Ser Val Asn Pro His Asp Ile Thr Val Gly Pro Val Ala Lys Ser Ile
            340                 345                 350

Leu Gln Gln Ala Gly Val Glu Met Lys Ser Glu Phe Leu Ala Thr Lys
        355                 360                 365

Ala Lys Gln Phe Gln Arg Ser Gln Leu Val Leu Val Thr Lys Gly Phe
    370                 375                 380

Asn Leu Phe Cys Lys Tyr Ile Tyr His Val Leu Trp His Ser Glu Phe
385                 390                 395                 400

Pro Lys Pro Gln Ile Leu Lys His Ala Met Lys Glu Cys Leu Glu Lys
                405                 410                 415

Cys Ile Glu Gln Asn Ile Thr Ser Ile Ser Phe Pro Ala Leu Gly Thr
            420                 425                 430

Gly Asn Met Glu Ile Lys Lys Glu Thr Ala Ala Glu Ile Leu Phe Asp
        435                 440                 445

Glu Val Leu Thr Phe Ala Lys Asp His Val Lys His Gln Leu Thr Val

450                 455                 460

Lys Phe Val Ile Phe Pro Thr Asp Leu Glu Ile Tyr Lys Ala Phe Ser
465                 470                 475                 480

Ser Glu Met Ala Lys Arg Ser Lys Met Leu Ser Leu Asn Asn Tyr Ser
                485                 490                 495

Val Pro Gln Ser Thr Arg Glu Glu Lys Arg Glu Asn Gly Leu Glu Ala
            500                 505                 510

Arg Ser Pro Ala Ile Asn Leu Met Gly Phe Asn Val Glu Glu Met Tyr
        515                 520                 525

Glu Ala His Ala Trp Ile Gln Arg Ile Leu Ser Leu Gln Asn His His
    530                 535                 540

Ile Ile Glu Asn Asn His Ile Leu Tyr Leu Gly Arg Lys Glu His Asp
545                 550                 555                 560

Ile Leu Ser Gln Leu Gln Lys Thr Ser Ser Val Ser Ile Thr Glu Ile
                565                 570                 575

Ile Ser Pro Gly Arg Thr Glu Leu Glu Ile Glu Gly Ala Arg Ala Asp
            580                 585                 590

Leu Ile Glu Val Val Met Asn Ile Glu Asp Met Leu Cys Lys Val Gln
        595                 600                 605

Glu Glu Met Ala Arg Lys Lys Glu Arg Gly Leu Trp Arg Ser Leu Gly
    610                 615                 620

Gln Trp Thr Ile Gln Gln Gln Lys Thr Gln Asp Glu Met Lys Glu Asn
625                 630                 635                 640

Ile Ile Phe Leu Lys Cys Pro Val Pro Pro Thr Gln Glu Leu Leu Asp
                645                 650                 655

Gln Lys Lys Gln Phe Glu Lys Cys Gly Leu Gln Val Leu Lys Val Glu
            660                 665                 670

Lys Ile Asp Asn Glu Val Leu Met Ala Ala Phe Gln Arg Lys Lys Lys
        675                 680                 685

Met Met Glu Glu Lys Leu His Arg Gln Pro Val Ser His Arg Leu Phe
    690                 695                 700

Gln Gln Val Pro Tyr Gln Phe Cys Asn Val Val Cys Arg Val Gly Phe
705                 710                 715                 720

Gln Arg Met Tyr Ser Thr Pro Cys Asp Pro Lys Tyr Gly Ala Gly Ile
                725                 730                 735

Tyr Phe Thr Lys Asn Leu Lys Asn Leu Ala Glu Lys Ala Lys Lys Ile
            740                 745                 750

Ser Ala Ala Asp Lys Leu Ile Tyr Val Phe Glu Ala Glu Val Leu Thr
        755                 760                 765

Gly Phe Phe Cys Gln Gly His Pro Leu Asn Ile Val Pro Pro Pro Leu
    770                 775                 780

Ser Pro Gly Ala Ile Asp Gly His Asp Ser Val Val Asp Asn Val Ser
785                 790                 795                 800

Ser Pro Glu Thr Phe Val Ile Phe Ser Gly Met Gln Ala Ile Pro Gln
                805                 810                 815

Tyr Leu Trp Thr Cys Thr Gln Glu Tyr Val Gln Ser Gln Asp Tyr Ser
            820                 825                 830

Ser Gly Pro Met Arg Pro Phe Ala Gln His Pro Trp Arg Gly Phe Ala
        835                 840                 845

Ser Gly Ser Pro Val Asp
    850

<210>  3
<211>  369
<212>  PRT
<213>  Homo sapiens

<400>  3

Met Ser Ser Arg Gly Gly Lys Lys Lys Ser Thr Lys Thr Ser Arg Ser
1               5                   10                  15

Ala Lys Ala Gly Val Ile Phe Pro Val Gly Arg Met Leu Arg Tyr Ile
            20                  25                  30

Lys Lys Gly His Pro Lys Tyr Arg Ile Gly Val Gly Ala Pro Val Tyr
        35                  40                  45

Met Ala Ala Val Leu Glu Tyr Leu Thr Ala Glu Ile Leu Glu Leu Ala
    50                  55                  60

Gly Asn Ala Ala Arg Asp Asn Lys Lys Gly Arg Val Thr Pro Arg His
65                  70                  75                  80

Ile Leu Leu Ala Val Ala Asn Asp Glu Glu Leu Asn Gln Leu Leu Lys
                85                  90                  95

Gly Val Thr Ile Ala Ser Gly Gly Val Leu Pro Asn Ile His Pro Glu
            100                 105                 110

Leu Leu Ala Lys Lys Arg Gly Ser Lys Gly Lys Leu Glu Ala Ile Ile
        115                 120                 125

Thr Pro Pro Pro Ala Lys Lys Ala Lys Ser Pro Ser Gln Lys Lys Pro
    130                 135                 140

Val Ser Lys Lys Ala Gly Gly Lys Lys Gly Ala Arg Lys Ser Lys Lys
145                 150                 155                 160

Lys Gln Gly Glu Val Ser Lys Ala Ala Ser Ala Asp Ser Thr Thr Glu
                165                 170                 175

Gly Thr Pro Ala Asp Gly Phe Thr Val Leu Ser Thr Lys Ser Leu Phe
            180                 185                 190

Leu Gly Gln Lys Leu Gln Val Val Gln Ala Asp Ile Ala Ser Ile Asp
        195                 200                 205

Ser Asp Ala Val Val His Pro Thr Asn Thr Asp Phe Tyr Ile Gly Gly
    210                 215                 220

Glu Val Gly Asn Thr Leu Glu Lys Lys Gly Gly Lys Glu Phe Val Glu
225                 230                 235                 240

Ala Val Leu Glu Leu Arg Lys Lys Asn Gly Pro Leu Glu Val Ala Gly
                245                 250                 255

Ala Ala Val Ser Ala Gly His Gly Leu Pro Ala Lys Phe Val Ile His
            260                 265                 270

Cys Asn Ser Pro Val Trp Gly Ala Asp Lys Cys Glu Glu Leu Leu Glu
        275                 280                 285

Lys Thr Val Lys Asn Cys Leu Ala Leu Ala Asp Asp Lys Lys Leu Lys
    290                 295                 300

Ser Ile Ala Phe Pro Ser Ile Gly Ser Gly Arg Asn Gly Phe Pro Lys
305                 310                 315                 320

Gln Thr Ala Ala Gln Leu Ile Leu Lys Ala Ile Ser Ser Tyr Phe Val
                325                 330                 335

Ser Thr Met Ser Ser Ser Ile Lys Thr Val Tyr Phe Val Leu Phe Asp
            340                 345                 350

Ser Glu Ser Ile Gly Ile Tyr Val Gln Glu Met Ala Lys Leu Asp Ala
        355                 360                 365

Asn

<210>  4
<211>  372
<212>  PRT
<213>  Homo sapiens

<400>  4

<210>  5
<211>  372
<212>  PRT
<213>  Homo sapiens

<400>  5

Met Ser Gly Arg Ser Gly Lys Lys Lys Met Ser Lys Leu Ser Arg Ser
1               5                   10                  15

Ala Arg Ala Gly Val Ile Phe Pro Val Gly Arg Leu Met Arg Tyr Leu
            20                  25                  30

Lys Lys Gly Thr Phe Lys Tyr Arg Ile Ser Val Gly Ala Pro Val Tyr
        35                  40                  45

Met Ala Ala Val Ile Glu Tyr Leu Ala Ala Glu Ile Leu Glu Leu Ala
    50                  55                  60

Gly Asn Ala Ala Arg Asp Asn Lys Lys Ala Arg Ile Ala Pro Arg His
65                  70                  75                  80

Ile Leu Leu Ala Val Ala Asn Asp Glu Glu Leu Asn Gln Leu Leu Lys
                85                  90                  95

Gly Val Thr Ile Ala Ser Gly Gly Val Leu Pro Arg Ile His Pro Glu
            100                 105                 110

Leu Leu Ala Lys Lys Arg Gly Thr Lys Gly Lys Ser Glu Thr Ile Leu
        115                 120                 125

Ser Pro Pro Pro Glu Lys Arg Gly Arg Lys Ala Thr Ser Gly Lys Lys
    130                 135                 140

Gly Gly Lys Lys Ser Lys Ala Ala Lys Pro Arg Thr Ser Lys Lys Ser
145                 150                 155                 160

Lys Pro Lys Asp Ser Asp Lys Glu Gly Thr Ser Asn Ser Thr Ser Glu
                165                 170                 175

Asp Gly Pro Gly Asp Gly Phe Thr Ile Leu Ser Ser Lys Ser Leu Val
            180                 185                 190

Leu Gly Gln Lys Leu Ser Leu Thr Gln Ser Asp Ile Ser His Ile Gly
        195                 200                 205

Ser Met Arg Val Glu Gly Ile Val His Pro Thr Thr Ala Glu Ile Asp
    210                 215                 220

Leu Lys Glu Asp Ile Gly Lys Ala Leu Glu Lys Ala Gly Gly Lys Glu
225                 230                 235                 240

Phe Leu Glu Thr Val Lys Glu Leu Arg Lys Ser Gln Gly Pro Leu Glu
                245                 250                 255

Val Ala Glu Ala Ala Val Ser Gln Ser Ser Gly Leu Ala Ala Lys Phe
            260                 265                 270

Val Ile His Cys His Ile Pro Gln Trp Gly Ser Asp Lys Cys Glu Glu
        275                 280                 285

Gln Leu Glu Glu Thr Ile Lys Asn Cys Leu Ser Ala Ala Glu Asp Lys
    290                 295                 300

Lys Leu Lys Ser Val Ala Phe Pro Pro Phe Pro Ser Gly Arg Asn Cys
305                 310                 315                 320

Phe Pro Lys Gln Thr Ala Ala Gln Val Thr Leu Lys Ala Ile Ser Ala
                325                 330                 335

His Phe Asp Asp Ser Ser Ala Ser Ser Leu Lys Asn Val Tyr Phe Leu
            340                 345                 350

Leu Phe Asp Ser Glu Ser Ile Gly Ile Tyr Val Gln Glu Met Ala Lys
        355                 360                 365

Leu Asp Ala Lys
    370

<210>  6
<211>  1872
<212>  PRT
<213>  Homo sapiens

<400>  6

Met Gly Pro Gly Cys Asp Leu Leu Leu Arg Thr Ala Ala Thr Ile Thr
1               5                   10                  15

Ala Ala Ala Ile Met Ser Asp Thr Asp Ser Asp Glu Asp Ser Ala Gly
            20                  25                  30

Gly Gly Pro Phe Ser Leu Ala Gly Phe Leu Phe Gly Asn Ile Asn Gly
        35                  40                  45

Ala Gly Gln Leu Glu Gly Glu Ser Val Leu Asp Asp Glu Cys Lys Lys
    50                  55                  60

His Leu Ala Gly Leu Gly Ala Leu Gly Leu Gly Ser Leu Ile Thr Glu
65                  70                  75                  80

Leu Thr Ala Asn Glu Glu Leu Thr Gly Thr Asp Gly Ala Leu Val Asn
                85                  90                  95

Asp Glu Gly Trp Val Arg Ser Thr Glu Asp Ala Val Asp Tyr Ser Asp
            100                 105                 110

Ile Asn Glu Val Ala Glu Asp Glu Ser Arg Arg Tyr Gln Gln Thr Met
        115                 120                 125

Gly Ser Leu Gln Pro Leu Cys His Ser Asp Tyr Asp Glu Asp Asp Tyr
    130                 135                 140

Asp Ala Asp Cys Glu Asp Ile Asp Cys Lys Leu Met Pro Pro Pro Pro
145                 150                 155                 160

Pro Pro Pro Gly Pro Met Lys Lys Asp Lys Asp Gln Asp Ser Ile Thr
                165                 170                 175

Gly Glu Lys Val Asp Phe Ser Ser Ser Ser Asp Ser Glu Ser Glu Met
            180                 185                 190

Gly Pro Gln Glu Ala Thr Gln Ala Glu Ser Glu Asp Gly Lys Leu Thr
        195                 200                 205

Leu Pro Leu Ala Gly Ile Met Gln His Asp Ala Thr Lys Leu Leu Pro
    210                 215                 220

Ser Val Thr Glu Leu Phe Pro Glu Phe Arg Pro Gly Lys Val Leu Arg
225                 230                 235                 240

Phe Leu Arg Leu Phe Gly Pro Gly Lys Asn Val Pro Ser Val Trp Arg
                245                 250                 255

Ser Ala Arg Arg Lys Arg Lys Lys Lys His Arg Glu Leu Ile Gln Glu
            260                 265                 270

Glu Gln Ile Gln Glu Val Glu Cys Ser Val Glu Ser Glu Val Ser Gln
        275                 280                 285

Lys Ser Leu Trp Asn Tyr Asp Tyr Ala Pro Pro Pro Pro Pro Glu Gln
    290                 295                 300

Cys Leu Ser Asp Asp Glu Ile Thr Met Met Ala Pro Val Glu Ser Lys
305                 310                 315                 320

Phe Ser Gln Ser Thr Gly Asp Ile Asp Lys Val Thr Asp Thr Lys Pro
                325                 330                 335

Arg Val Ala Glu Trp Arg Tyr Gly Pro Ala Arg Leu Trp Tyr Asp Met

            340                 345                 350

Leu Gly Val Pro Glu Asp Gly Ser Gly Phe Asp Tyr Gly Phe Lys Leu
        355                 360                 365

Arg Lys Thr Glu His Glu Pro Val Ile Lys Ser Arg Met Ile Glu Glu
    370                 375                 380

Phe Arg Lys Leu Glu Glu Asn Asn Gly Thr Asp Leu Leu Ala Asp Glu
385                 390                 395                 400

Asn Phe Leu Met Val Thr Gln Leu His Trp Glu Asp Asp Ile Ile Trp
                405                 410                 415

Asp Gly Glu Asp Val Lys His Lys Gly Thr Lys Pro Gln Arg Ala Ser
            420                 425                 430

Leu Ala Gly Trp Leu Pro Ser Ser Met Thr Arg Asn Ala Met Ala Tyr
        435                 440                 445

Asn Val Gln Gln Gly Phe Ala Ala Thr Leu Asp Asp Asp Lys Pro Trp
    450                 455                 460

Tyr Ser Ile Phe Pro Ile Asp Asn Glu Asp Leu Val Tyr Gly Arg Trp
465                 470                 475                 480

Glu Asp Asn Ile Ile Trp Asp Ala Gln Ala Met Pro Arg Leu Leu Glu
                485                 490                 495

Pro Pro Val Leu Thr Leu Asp Pro Asn Asp Glu Asn Leu Ile Leu Glu
            500                 505                 510

Ile Pro Asp Glu Lys Glu Glu Ala Thr Ser Asn Ser Pro Ser Lys Glu
        515                 520                 525

Ser Lys Lys Glu Ser Ser Leu Lys Lys Ser Arg Ile Leu Leu Gly Lys
    530                 535                 540

Thr Gly Val Ile Lys Glu Glu Pro Gln Gln Asn Met Ser Gln Pro Glu
545                 550                 555                 560

Val Lys Asp Pro Trp Asn Leu Ser Asn Asp Glu Tyr Tyr Tyr Pro Lys
                565                 570                 575

Gln Gln Gly Leu Arg Gly Thr Phe Gly Gly Asn Ile Ile Gln His Ser
            580                 585                 590

Ile Pro Ala Val Glu Leu Arg Gln Pro Phe Phe Pro Thr His Met Gly
        595                 600                 605

Pro Ile Lys Leu Arg Gln Phe His Arg Pro Pro Leu Lys Lys Tyr Ser
    610                 615                 620

Phe Gly Ala Leu Ser Gln Pro Gly Pro His Ser Val Gln Pro Leu Leu
625                 630                 635                 640

Lys His Ile Lys Lys Lys Ala Lys Met Arg Glu Gln Glu Arg Gln Ala
                645                 650                 655

Ser Gly Gly Gly Glu Met Phe Phe Met Arg Thr Pro Gln Asp Leu Thr
            660                 665                 670

Gly Lys Asp Gly Asp Leu Ile Leu Ala Glu Tyr Ser Glu Glu Asn Gly
        675                 680                 685

Pro Leu Met Met Gln Val Gly Met Ala Thr Lys Ile Lys Asn Tyr Tyr
    690                 695                 700

Lys Arg Lys Pro Gly Lys Asp Pro Gly Ala Pro Asp Cys Lys Tyr Gly
705                 710                 715                 720

Glu Thr Val Tyr Cys His Thr Ser Pro Phe Leu Gly Ser Leu His Pro
                725                 730                 735

Gly Gln Leu Leu Gln Ala Phe Glu Asn Asn Leu Phe Arg Ala Pro Ile
            740                 745                 750

Tyr Leu His Lys Met Pro Glu Thr Asp Phe Leu Ile Ile Arg Thr Arg
        755                 760                 765

Gln Gly Tyr Tyr Ile Arg Glu Leu Val Asp Ile Phe Val Val Gly Gln
    770                 775                 780

Gln Cys Pro Leu Phe Glu Val Pro Gly Pro Asn Ser Lys Arg Ala Asn
785                 790                 795                 800

Thr His Ile Arg Asp Phe Leu Gln Val Phe Ile Tyr Arg Leu Phe Trp
                805                 810                 815

Lys Ser Lys Asp Arg Pro Arg Arg Ile Arg Met Glu Asp Ile Lys Lys
            820                 825                 830

Ala Phe Pro Ser His Ser Glu Ser Ser Ile Arg Lys Arg Leu Lys Leu
        835                 840                 845

Cys Ala Asp Phe Lys Arg Thr Gly Met Asp Ser Asn Trp Trp Val Leu
    850                 855                 860

Lys Ser Asp Phe Arg Leu Pro Thr Glu Glu Glu Ile Arg Ala Met Val
865                 870                 875                 880

Ser Pro Glu Gln Cys Cys Ala Tyr Tyr Ser Met Ile Ala Ala Glu Gln
                885                 890                 895

Arg Leu Lys Asp Ala Gly Tyr Gly Glu Lys Ser Phe Phe Ala Pro Glu
            900                 905                 910

Glu Glu Asn Glu Glu Asp Phe Gln Met Lys Ile Asp Asp Glu Val Arg
        915                 920                 925

Thr Ala Pro Trp Asn Thr Thr Arg Ala Phe Ile Ala Ala Met Lys Gly
    930                 935                 940

Lys Cys Leu Leu Glu Val Thr Gly Val Ala Asp Pro Thr Gly Cys Gly
945                 950                 955                 960

Glu Gly Phe Ser Tyr Val Lys Ile Pro Asn Lys Pro Thr Gln Gln Lys
                965                 970                 975

Asp Asp Lys Glu Pro Gln Pro Val Lys Lys Thr Val Thr Gly Thr Asp
            980                 985                 990

Ala Asp Leu Arg Arg Leu Ser Leu  Lys Asn Ala Lys Gln  Leu Leu Arg
        995                 1000                1005

Lys Phe  Gly Val Pro Glu Glu  Glu Ile Lys Lys Leu  Ser Arg Trp
    1010                 1015                 1020

Glu Val  Ile Asp Val Val Arg  Thr Met Ser Thr Glu  Gln Ala Arg
    1025                 1030                 1035

Ser Gly  Glu Gly Pro Met Ser  Lys Phe Ala Arg Gly  Ser Arg Phe
    1040                 1045                 1050

Ser Val  Ala Glu His Gln Glu  Arg Tyr Lys Glu Glu  Cys Gln Arg
    1055                 1060                 1065

Ile Phe  Asp Leu Gln Asn Lys  Val Leu Ser Ser Thr  Glu Val Leu
    1070                 1075                 1080

Ser Thr  Asp Thr Asp Ser Ser  Ser Ala Glu Asp Ser  Asp Phe Glu
    1085                 1090                 1095

Glu Met Gly Lys Asn Ile Glu Asn Met Leu Gln Asn Lys Lys Thr
    1100                1105                1110

Ser Ser Gln Leu Ser Arg Glu Arg Glu Glu Gln Glu Arg Lys Glu
    1115                1120                1125

Leu Gln Arg Met Leu Leu Ala Ala Gly Ser Ala Ala Ser Gly Asn
    1130                1135                1140

Asn His Arg Asp Asp Asp Thr Ala Ser Val Thr Ser Leu Asn Ser
    1145                1150                1155

Ser Ala Thr Gly Arg Cys Leu Lys Ile Tyr Arg Thr Phe Arg Asp
    1160                1165                1170

Glu Glu Gly Lys Glu Tyr Val Arg Cys Glu Thr Val Arg Lys Pro
    1175                1180                1185

Ala Val Ile Asp Ala Tyr Val Arg Ile Arg Thr Thr Lys Asp Glu
    1190                1195                1200

Glu Phe Ile Arg Lys Phe Ala Leu Phe Asp Glu Gln His Arg Glu
    1205                1210                1215

Glu Met Arg Lys Glu Arg Arg Arg Ile Gln Glu Gln Leu Arg Arg
    1220                1225                1230

Leu Lys Arg Asn Gln Glu Lys Glu Lys Leu Lys Gly Pro Pro Glu
    1235                1240                1245

Lys Lys Pro Lys Lys Met Lys Glu Arg Pro Asp Leu Lys Leu Lys
    1250                1255                1260

Cys Gly Ala Cys Gly Ala Ile Gly His Met Arg Thr Asn Lys Phe
    1265                1270                1275

Cys Pro Leu Tyr Tyr Gln Thr Asn Ala Pro Pro Ser Asn Pro Val
    1280                1285                1290

Ala Met Thr Glu Glu Gln Glu Glu Glu Leu Glu Lys Thr Val Ile
    1295                1300                1305

His Asn Asp Asn Glu Glu Leu Ile Lys Val Glu Gly Thr Lys Ile
    1310                1315                1320

Val Leu Gly Lys Gln Leu Ile Glu Ser Ala Asp Glu Val Arg Arg

1325                1330                1335

Lys Ser Leu Val Leu Lys Phe Pro Lys Gln Gln Leu Pro Pro Lys
    1340                1345                1350

Lys Lys Arg Arg Val Gly Thr Thr Val His Cys Asp Tyr Leu Asn
    1355                1360                1365

Arg Pro His Lys Ser Ile His Arg Arg Arg Thr Asp Pro Met Val
    1370                1375                1380

Thr Leu Ser Ser Ile Leu Glu Ser Ile Ile Asn Asp Met Arg Asp
    1385                1390                1395

Leu Pro Asn Thr Tyr Pro Phe His Thr Pro Val Asn Ala Lys Val
    1400                1405                1410

Val Lys Asp Tyr Tyr Lys Ile Ile Thr Arg Pro Met Asp Leu Gln
    1415                1420                1425

Thr Leu Arg Glu Asn Val Arg Lys Arg Leu Tyr Pro Ser Arg Glu
    1430                1435                1440

Glu Phe Arg Glu His Leu Glu Leu Ile Val Lys Asn Ser Ala Thr
    1445                1450                1455

Tyr Asn Gly Pro Lys His Ser Leu Thr Gln Ile Ser Gln Ser Met
    1460                1465                1470

Leu Asp Leu Cys Asp Glu Lys Leu Lys Glu Lys Glu Asp Lys Leu
    1475                1480                1485

Ala Arg Leu Glu Lys Ala Ile Asn Pro Leu Leu Asp Asp Asp Asp
    1490                1495                1500

Gln Val Ala Phe Ser Phe Ile Leu Asp Asn Ile Val Thr Gln Lys
    1505                1510                1515

Met Met Ala Val Pro Asp Ser Trp Pro Phe His His Pro Val Asn
    1520                1525                1530

Lys Lys Phe Val Pro Asp Tyr Tyr Lys Val Ile Val Asn Pro Met
    1535                1540                1545

Asp Leu Glu Thr Ile Arg Lys Asn Ile Ser Lys His Lys Tyr Gln
    1550                1555                1560

Ser Arg Glu Ser Phe Leu Asp Asp Val Asn Leu Ile Leu Ala Asn
    1565                1570                1575

Ser Val Lys Tyr Asn Gly Pro Glu Ser Gln Tyr Thr Lys Thr Ala
    1580                1585                1590

Gln Glu Ile Val Asn Val Cys Tyr Gln Thr Leu Thr Glu Tyr Asp
    1595                1600                1605

Glu His Leu Thr Gln Leu Glu Lys Asp Ile Cys Thr Ala Lys Glu
    1610                1615                1620

Ala Ala Leu Glu Glu Ala Glu Leu Glu Ser Leu Asp Pro Met Thr
    1625                1630                1635

Pro Gly Pro Tyr Thr Pro Gln Pro Pro Asp Leu Tyr Asp Thr Asn
    1640                1645                1650

Thr Ser Leu Ser Met Ser Arg Asp Ala Ser Val Phe Gln Asp Glu
    1655                1660                1665

Ser Asn Met Ser Val Leu Asp Ile Pro Ser Ala Thr Pro Glu Lys
    1670                1675                1680

Gln Val Thr Gln Glu Gly Glu Asp Gly Asp Gly Asp Leu Ala Asp
    1685                1690                1695

Glu Glu Glu Gly Thr Val Gln Gln Pro Gln Ala Ser Val Leu Tyr
    1700                1705                1710

Glu Asp Leu Leu Met Ser Glu Gly Glu Asp Asp Glu Glu Asp Ala
    1715                1720                1725

Gly Ser Asp Glu Glu Gly Asp Asn Pro Phe Ser Ala Ile Gln Leu
    1730                1735                1740

Ser Glu Ser Gly Ser Asp Ser Asp Val Gly Ser Gly Gly Ile Arg
    1745                1750                1755

Pro Lys Gln Pro Arg Met Leu Gln Glu Asn Thr Arg Met Asp Met
    1760                1765                1770

Glu Asn Glu Glu Ser Met Met Ser Tyr Glu Gly Asp Gly Gly Glu
    1775                1780                1785

Ala Ser His Gly Leu Glu Asp Ser Asn Ile Ser Tyr Gly Ser Tyr
    1790                1795                1800

Glu Glu  Pro Asp Pro Lys Ser  Asn Thr Gln Asp Thr  Ser Phe Ser
    1805                 1810                 1815

Ser Ile  Gly Gly Tyr Glu Val  Ser Glu Glu Glu Glu  Asp Glu Glu
    1820                 1825                 1830

Glu Glu  Glu Gln Arg Ser Gly  Pro Ser Val Leu Ser  Gln Val His
    1835                 1840                 1845

Leu Ser  Glu Asp Glu Glu Asp  Ser Glu Asp Phe His  Ser Ile Ala
    1850                 1855                 1860

Gly Asp  Ser Asp Leu Asp Ser  Asp Glu
    1865                 1870